# EUROPEAN PATENT SPECIFICATION

(11) **EP 1 238 636 B1**
(45) Date of publication and mention of the grant of the patent: **30.08.2006**
(21) Application number: 01830149.9
(22) Date of filing: 05.03.2001
(51) Int. Cl.: A61B 17/64

(54) **External fixation device with identification means**
Äusserliche Fixierung mit Identifikationsmarke
Fixateur externe avec etiquette d'identification

(43) Date of publication of application: 11.09.2002
(73) Proprietor: ORTHOFIX INTERNATIONAL B.V., 1071 Amsterdam (NL)
(72) Inventor: Coati, Michele, 37029 San Pietro in Cariano (IT); Rossi, Luigi, 37019 Peschiera del Garda (IT)
(74) Representative: Zambardino, Umberto

(56) References cited:
- EP-A- 0 807 419
- WO-A-99/04719
- FR-A- 2 770 127
- US-A- 4 671 916
- US-A- 5 662 648
- US-A- 6 030 386

## Description

### Field of Application

This invention broadly relates to an external fixation device for reducing bone fractures in orthopedic surgery.

More particularly, the invention relates to an external fixation device having a carrying structure made up of interconnected elements, specifically a structure comprising an extendible central body and oppositely located clamps for bone screws, which clamps are articulated to respective ends of the central body by means of ball joints.

### Prior Art

In this specific technical field, external fixation devices have long been employed to foster recovery of bone fractures by holding the bone fragments firmly in place.

Such external fixation devices comprise a central body of substantially cylindrical shape, which is extendible axially and has bone screw clamps articulated to its opposed ends by means of ball joints. The clamps are connected to rod-like bone screws which have been implanted into the cortex of the broken bone, from either sides of the fracture. Usually, two or three screws are adequate to guarantee a broken bone.

A variety of fixation devices to cope with different types of fractures and traumatisms are normally available from their suppliers.

Thus, fixation devices for the tibia and the femur, fixation devices for the humerus, fixation devices for joints such as the ankle and the elbow, and fixation devices for the wrist are available.

A well-recognized demand in this technical field is that of providing the device with visually identifiable means which should assist the orthopedist in his/her work of placing the device to a fractured part of an individual's skeleton, and later, which should give a visual indication, even approximate, of the mutual positions of the working parts of the fixation device.

Another demand from this field is that of providing the fixation device with decorative elements effective to enhance the outward appearance of the fixation device, for the orthopedist's sake as well as the patient's sake.

Unfortunately, state-of-art fixation devices come short of filling the above demands in that they are made of a homogeneous structure, usually of a plastic material transparent to X-radiation to permit unobstructed radiographs of the fractured region. Thus, the outward appearance of such fixation devices is often so unsightly that they are hidden under some covering.

Furthermore, any improvements in the direction of filling the above demands are restrained by the relatively high manufacturing cost of fixation devices, especially those incorporating one or more elements made up of a material transparent to X-radiation, and by the need to maintain a desired transparency to X-radiation and biocompatibility of the used materials.

European patent EP 0 807 419 discloses a compact external fixator for the treatment of fractures in small bones and in children comprising clamps connected to the central body by means of corresponding ball joints, the central body comprising a male and a female member telescopically joined.

US patent A 4 671 916 discloses a method of identifying surgical instruments as belonging to a set, which comprises forming a bore in a suitable location of the instrument and inserting and securing at least one colored plug.

French patent FR 2 770 127 discloses a method for marking surgery instruments in which the marking element is inserted in the instrument, the marking element forming a larger head, complementary to the hole formed in the instrument, such method comprising the steps of inserting the marking element and cutting excess material raising from the surface so as to obtain a smooth surface.

The underlying technical problem of this invention is to provide an external fixation device for stabilizing bone fractures, with structural and functional features appropriate to fill at least one of the aforesaid demands.

### Summary of the Invention

The technical problem is solved according to the invention by an external fixation device as previously indicated and as defined in the characterizing portion of Claim 1 and followings.

The features and advantages of the external fixation device according to the invention will be apparent from the following description of embodiments thereof, given by way of non-limitative examples with reference to the accompanying drawings.

### Brief Description of the Drawings

In the drawings:
Figure 1 is a perspective view of a unilateral external fixation device according to this invention, shown in its closed setting;
Figure 2 is a perspective view of the fixation device in Figure 1, shown in its open setting;
Figure 3 is an exploded perspective view of the fixation device shown in Figure 1;
Figures 4 to 6 are side views, taken under different angles, of the fixation device shown in Figure 2;
Figure 7 is an unilateral axial type of external fixation device according to another embodiment of this invention;
Figures 7a and 7b are side views, taken under different angles, of the fixation device shown in Figure 7;
Figure 8 is a perspective view of a detail of the fixation device shown in Figure 8;
Figure 9 is an exploded perspective view of the detail shown in Figure 8;
Figure 10 is a perspective view of another detail of the fixation device shown in Figure 7; and
Figure 11 is an exploded perspective view of the detail shown in Figure 10.

### Detailed Description

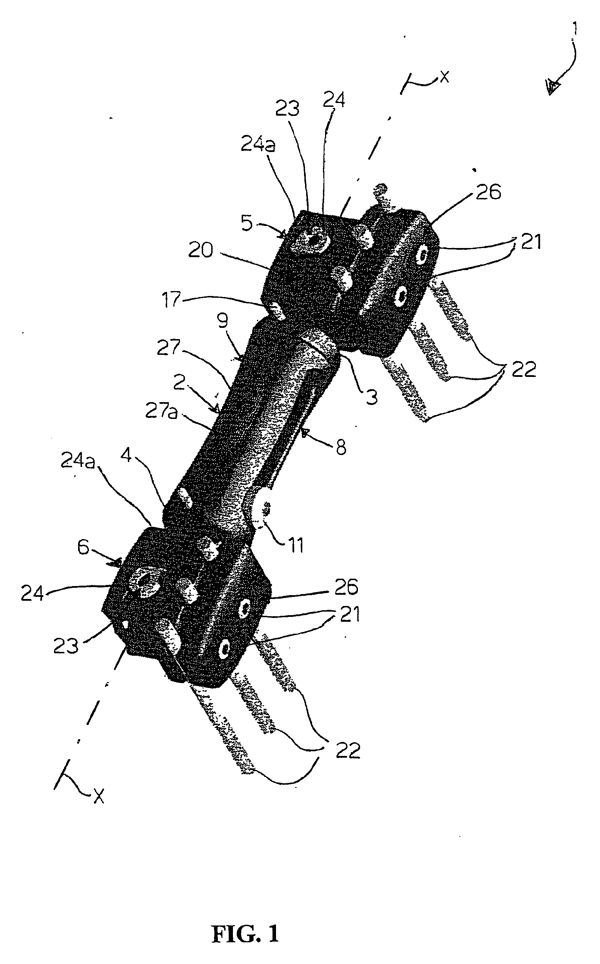

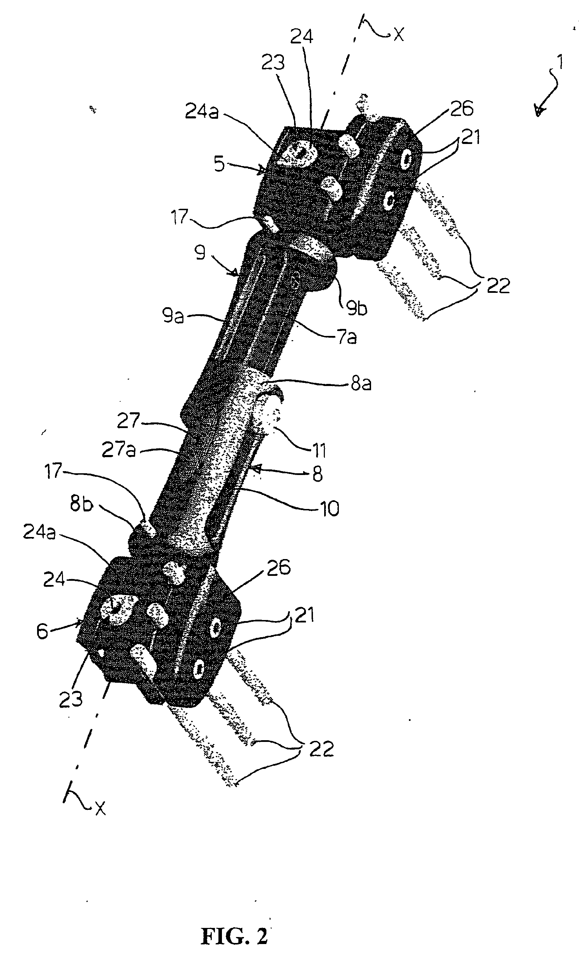

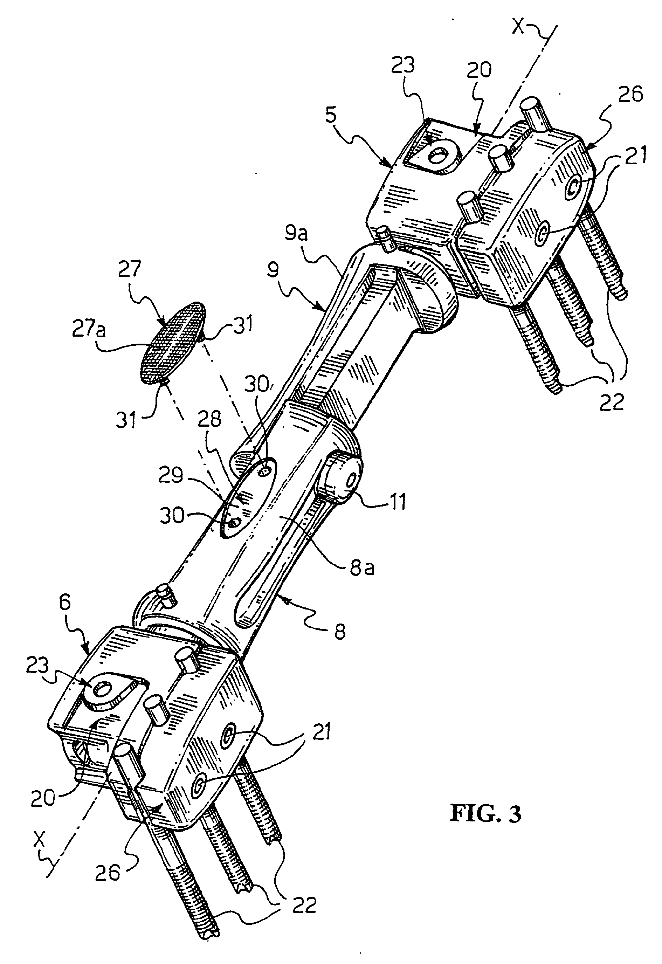

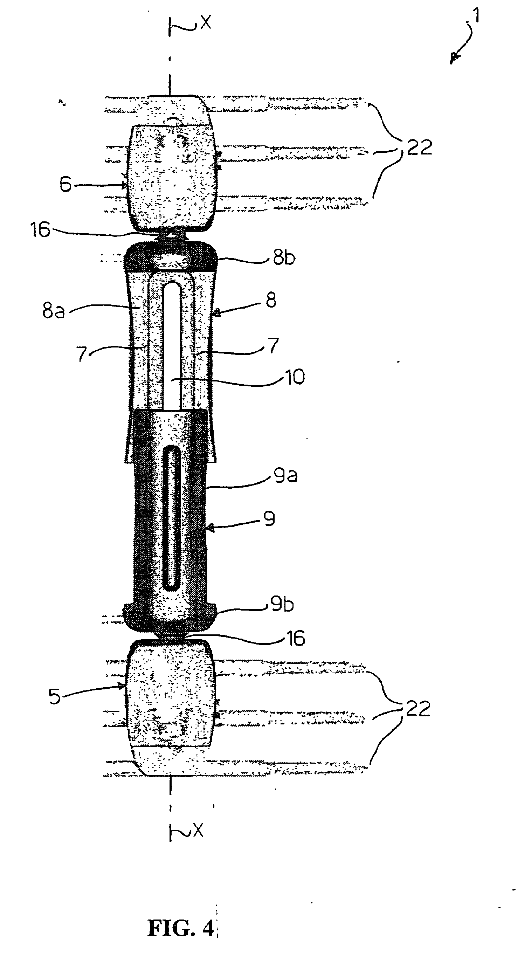

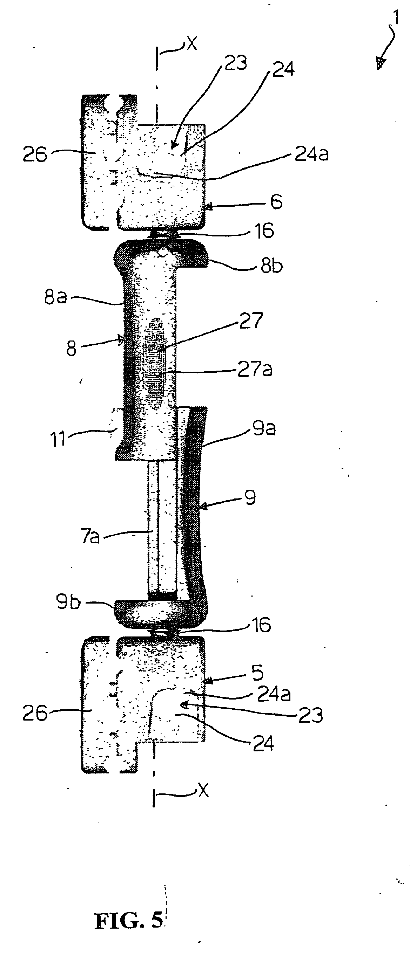

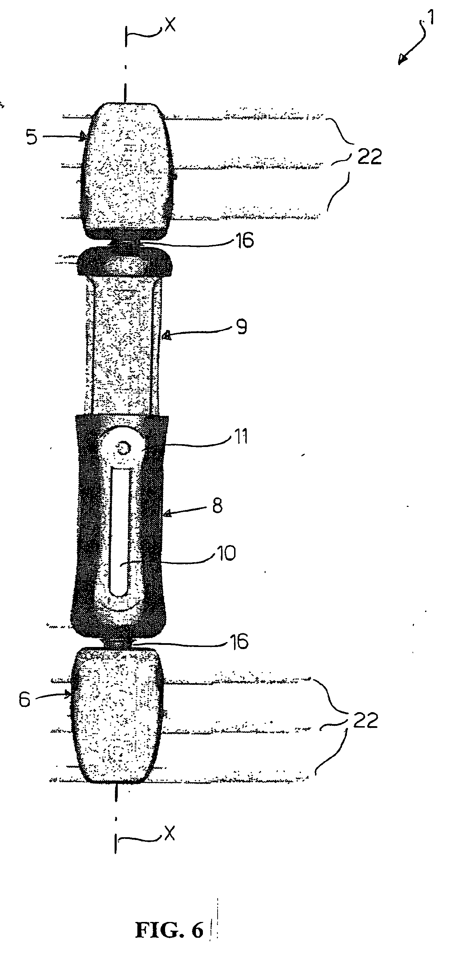

With reference to annexed Figures 1 to 6, a unilateral axial external fixation device for reducing bone fractures in orthopedic surgery, according to this invention, is shown generally at 1.

The fixation device 1 has a carrying structure made up of interconnected elements, which structure comprises a rod-like central body 2 having a longitudinal axis x-x, and having opposite ends 3, 4 which are articulated to respective clamps 5, 6 for bone screws.

The rod-like central body 2 and clamps 5, 6 are preferably made up of a material transparent to X-radiation, such as a polyetherketone plastics matrix, known as "Peek", suitably reinforced with a predetermined amount of carbon fibers to confer a suitable rigidity.

The rod-like central body 2 is axially extendible and comprises a first part 8 and a second part 9 slidably coupled to each other together to allow the central body 2 to be extended telescopically.

Said parts 8, 9 comprise each a first portion 8a, 9a, of elongate shape, which is formed integrally with a second end portion 8b, 9b being substantially cylindrical in shape and shorter. Thus, each part 8, 9 is L-shaped in side view.

The elongate portions 8a, 9a are slidably coupled to each other by means of a driving groove 7, formed longitudinally in the portion 8a, and a corresponding slide 7a formed longitudinally in the other portion 9a.

The fixation device 1 further comprises conventional means for stopping said parts 8 and 9 in their sliding movement. This stopping means consists of a set screw 11 extending perpendicularly to the longitudinal axis x-x and having a threaded end portion screwed in a conventional manner in a threaded hole (not shown) provided in the slide 7a.

In addition, the portion 8a of the part 8 is formed with a clearance slot 10 which spans longitudinally most of said portion to let the screw 11 go through it preferably with a smooth section of its shank for easier sliding movement of the portions 8 and 9 one on the other.

The screw 11 is an Allen screw, for more convenient operation by a corresponding wrench.

The extension of the rod-like central body 2 of the fixation device 1 can be adjusted by loosening and tightening the screw 11, depending from the dimensions of the broken bone. No relative rotation is instead allowed of the parts 8 and 9 about the longitudinal axis x-x of the rod-like central body 2, due to the C- and T-shaped cross-sections of the driving groove and the slide.

In the setting of minimum bulk of the rod-like central body 2, the elongate portions 8a and 9a of the parts 8 and 9 result fully superposed, with one abutting the cylindrical end portion 9b, 8b of the other.

The opposed ends 3, 4 of the rod-like central body 2 are articulated to the respective clamps 5, 6 for bone screws by means of ball joints.

These ball joints are known *per se,* and are ball-and-socket joints 16 mounted to each clamp 5, 6.

In particular, each ball-and-socket joint 16 comprises a ball head with a shank, the ball head being fitted into a socket formed in each clamp 5, 6, and the shank being fitted, in a conventional manner, in a corresponding seat in each of the ends 3, 4 of the rod-like central body 2.

The shank is held in its seat by a conventional releasable retention means, e.g. a pin 17. This pin 17 also provides a reference base for a dynamization compressor, such as that disclosed in European Patent Application No. 0 734 233.

The clamps 5 and 6 comprise each a main body 20, in which the socket for receiving the ball head of the ball-and-socket joint 16 is formed.

A sideplate 26 is removably associated with the main body 20 for tightening the clamp onto bone screws 22, in the example three screws 22 lying in a plane parallel to the plane containing the axis x-x and being placed a predetermined distance away therefrom.

The fixation device 1 also comprises locking means 23 of the ball-and-socket joint 16 in a selected angular position. This locking means 23 comprises of a drive rotary shaft acting in the main body 20 and having a handling end 24 that protrudes from said main body 20.

The end 24 has a radial projection 24a for more convenient manipulation of the locking means 23.

The structural and operational characteristics of the clamps 5 and 6, of the locking means 23, of the sideplate 26, and of the releasable connection of the latter to the main body 20 are conventional, and will not be further discussed.

In accordance with the invention, the fixation device 1 has an oval plate 27 of a given thickness releasably attached to the rod-like central body 2.

In particular, the plate 27 is lodged into a corresponding seat consisting of a recess 28 formed on the surface of the elongate portion 8a of the rod-like central body 2. Preferably, the depth of the recess 28 is substantially equal to the thickness of the plate 27.

The plate 27 is made releasably fast with the elongate portion 8a, by fitting two opposed pins 31 integrally formed on the underside of the plate 27 into corresponding blind holes 30 formed in the bottom 29 of the recess 28.

Thus, the plate 27 is secured in the recess 28, but is easily removed for replacement.

Advantageously, the plate 27 is formed from a plastic material that is transparent to X-radiation and a contrasting color with that of the rod-like body 2, so that the chromatic effect will distinguish the fixation device 1 of this invention.

However, this plate could be made of another plastics or aluminum alloy material producing low interference on X-radiation.

The plastic plate 27 is formed by conventional processes such as injection molding, and can be either bulk-stained or post-molding dyed by conventional methods.

Preferably, the plate 27 is made with a thin thickness, preferably comprised in the range of 0.5 to 1.5 mm, so that the plate will not appreciably interfere on a standard X-raying apparatus as a result of any opaque material to X-radiation entering its composition and/or because of the dyestuff.

Also, the plate 27 can bear, on its outer surface 27a exposed to view, such captions, insignia, logos and/or graphic compositions as may be the manufacturer's choice and/or demanded by the user, for example, in order to distinguish the fixation device 1 according to specific taste and personal preference.

In this embodiment, the visible outer surface 27a of the plate 27 is advantageously knurled to render more safe the grip and the manipulation of the fixation device 1.

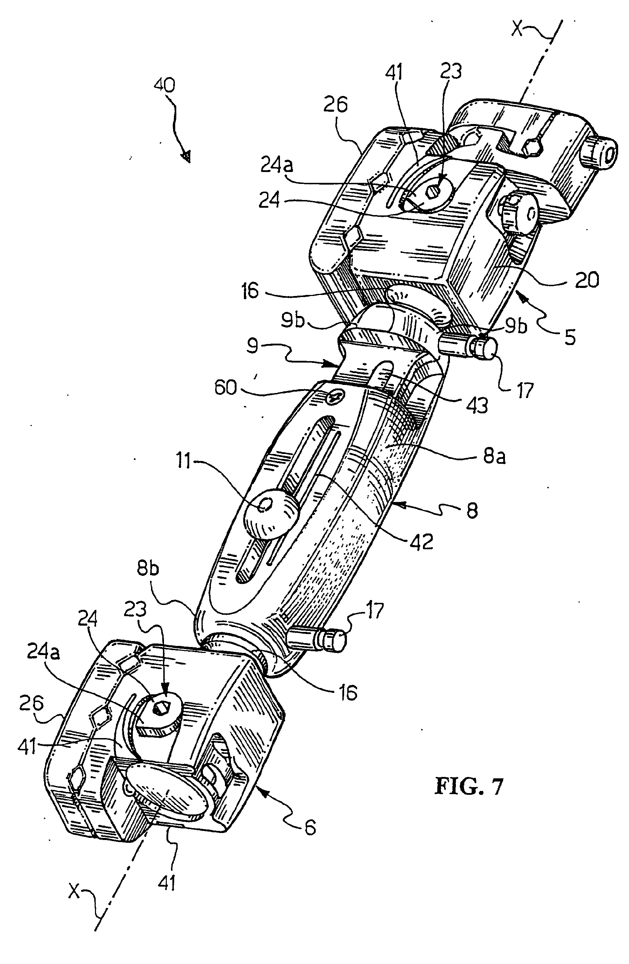

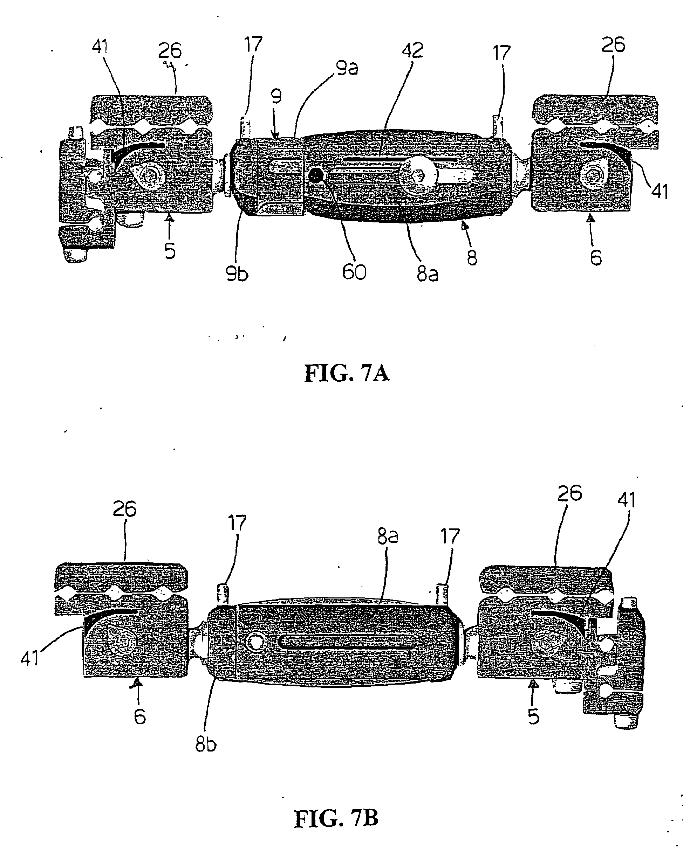

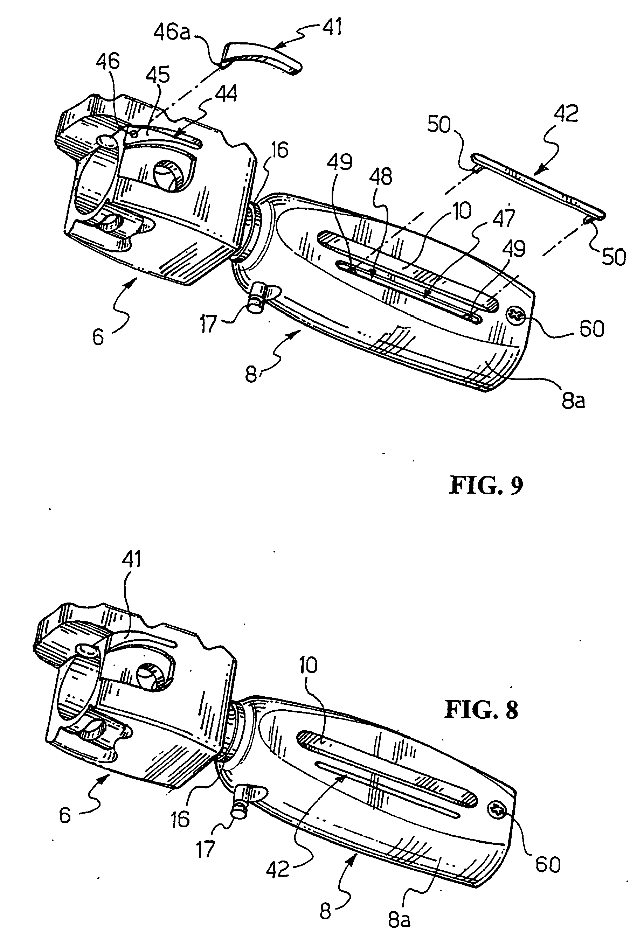

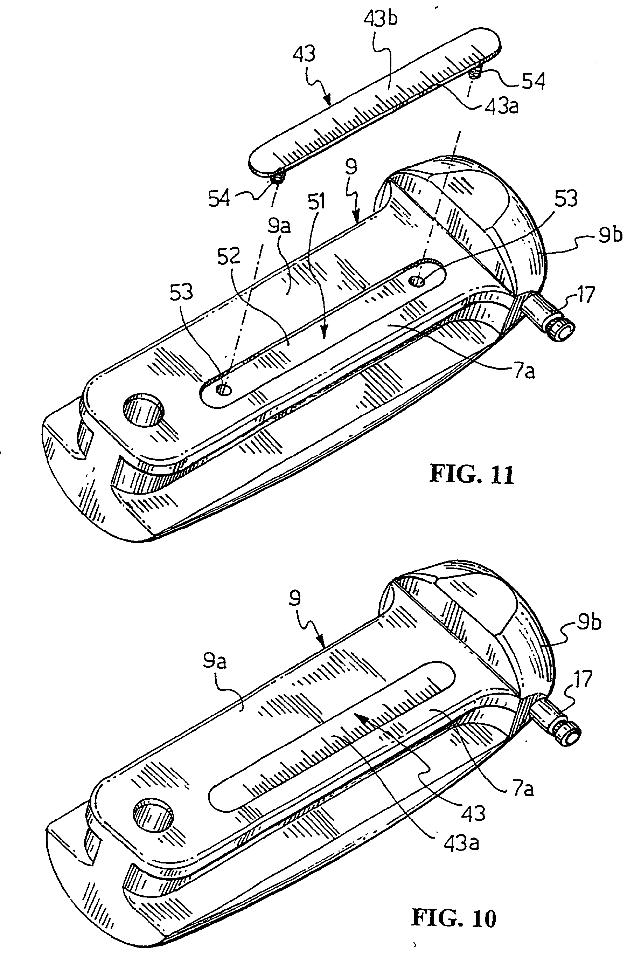

Making now reference to Figures 7 to 11, an external fixation device of the unilateral axial type, according to another embodiment of the invention, is shown generally at 40.

In all these Figures, elements having the same construction as or functionally equivalent to those of the fixation device 1 shown in Figures 1 to 6 are denoted by the same reference numerals.

The fixation device 40 has two plates 41 releasably attached to opposed sides of each clamp 5, 6, two plates 42 and 60 releasably attached to part 8 of the rod-like central body 2, and one plate 43 releasably attached to part 9 of the central body 2.

In particular, each plate 41 is placed close to the locking means 23 of the ball-and-socket joint 16 and extends between a substantially central location on one of the clamps 5, 6 and the free end thereof, so as to provide a visual indication of the movement of the radial projection 24a on the handling end 24 of said locking means 23.

In other words, each plate 41 situates in the background from the arcuate path travelled by the radial projection 24a as the end 24 of the locking means 23 is manipulated.

In practice, each plate 41 has a substantially comma-shaped contour with a cross-section that decreases gradually toward a central position of one of the clamps 5, 6.

Thus, each plate 41 advantageously provides the orthopedist with a visual indication of the rotation of the handling end 24, as well as a visual indication, albeit approximate, of the operational state of the locking means 23.

For example, a radial projection 24a aligned to regions of increasing cross-section of the plate 41 as a result of the handling end 24 having been manipulated, advantageously provides the orthopedist with an indication of increased effectiveness of the ball-and-socket joint 16 locking.

Each plate 41 is lodged in a respective seat in one of the clamps 5, 6. This seat consists of a recess 44 having preferably a depth from the surface that is substantially equal to the thickness of a plate 41.

Each plate 41 is releasably fast to one of the clamps 5, 6 by fitting a pin 46a, integrally formed on the underside of the plate 41 into a corresponding blind hole 46 formed in the bottom 45 of a recess 44.

The plate 42 consists of an elongate bar extending lengthwise on the elongate portion 8a of the rod-like central body 2, laterally of the slot 10, to nearly span its full axial length.

In practice, the plate 42 is lodged in a corresponding seat provided in the elongate portion 8a of the rod-like central body 2. This seat consists of a recess 47 having preferably a depth from the surface that is substantially equal to the thickness of the plate 42.

The plate 42 is releasably fast to the elongate portion 8a by fitting two opposed pins 50, integrally formed on the underside of the plate 42 into respective blind holes 49 provided in the bottom 48 of the recess 47.

The plate 60 is circular in shape and locates centrally close to one end of the elongate portion 8a of the rod-like central body 2. The releasable attachment of the plate 60 to the elongate portion 8a is performed in a similar way as the plate 42, and therefore it will not be further described.

The plate 43 consists of an elongate bar extending lengthwise on the slide 7a formed in the portion 9a of the rod-like central body 2.

In practice, the plate 43 is lodged in a corresponding seat provided in said slide 7a. This seat consists of a recess 51 having preferably a depth from the surface that is substantially equal to the thickness of the plate 43.

The plate 43 is releasably fast to the slide 7a by fitting two opposed pins 54 integrally formed on the underside of the plate 43 in respective blind holes 53 provided in the bottom 52 of the recess 51.

In particular, the plate 43 has a visible outer surface 43b impressed with a calibrated scale 43a that advantageously provides the orthopedist with a measure of the extension of the rod-like central body 2 achieved by a sliding movement of the parts 8 and 9.

The plates 41, 42, 43 and 60 just described are advantageously made of a plastic material transparent to X-radiation and colored to create contrast with the color of the fixation device parts bearing them. Alternatively, these plates could be made of any other plastics or aluminum alloy material producing low interference on X-radiation.

Preferably, the plates 41, 42, 43 and 60 are made with a thin thickness preferably comprised in the range of 0.5 to 1.5 mm, so that they will not interfere appreciably on standard X-ray equipment.

The fixation device of this invention does solve the technical problem and offers a number of advantages, among which the foremost is its being highly versatile and quite simple to manufacture. In fact, according to the invention, any decorative element can be provided on the fixation device structure by exchanging plates of contrasting colors, rendering the plates interchangeable.

Another advantage of the fixation device according to the invention is that the plates can be made of the same material transparent to X-radiation as that of the other components of the device, so that the plates would not interfere on standard X-ray equipment, or would only interfere to a reduced extent by reason of the dyestuff they incorporate.

Furthermore, in the event of the plates being made of an plastic opaque to X-radiation or an aluminum alloy, their interference on the X-ray equipment can be suppressed or at least attenuated by making them very thin and/or placing them in unexposed areas of the device to X-radiation.

A further advantage of the fixation device according to the invention is that of its high level of bio-compatibility which is not altered by the presence of contrasting colour plates thereon.

Still another, no less important advantage of the fixation device according to the invention, is that the provision of decorating plates is of minor relevance to the manufacturing cost of the device. Consequently, the manufacturing cost of latter is fully comparable with that of similar fixation devices of the state of the art.

In order to satisfy contingent and specific requirements, a skilled person in the art could make, in a obvious way, several changes and modifications to the fixation device of this invention without departing from the scope of protection of the invention as defined by the following claims.

## Claims

1. An external fixation device for reducing bone fractures comprising a carrying structure (1;40) of interconnected elements (2,5,6), said interconnected elements (2,5,6) comprising an extendible central body (2), and clamps (5,6) for bone screws respectively articulated on opposed ends (3,4) of the central body (2), **characterized in that** it comprises at least one plate (27;41;42;43;60) having predetermined contour shape and thickness, at least one recess (28;44;47;51) formed on the surface of at least one of the interconnected elements (2,5,6) and having its contour shape matching the contour shape of said at least one plate (27;41;42;43;60), and means (30,31;46,46a;49,50;53,54) for releasably securing said at least one plate (27;41;42;43;60) in said at least one recess (28;44;47;51).

2. A fixation device according to Claim 1, in which the depth of said at least one recess (28;44;47;51) is substantially equal to the thickness of said at least one plate (27;41;42;43;60).

3. A fixation device according to either Claim 1 or Claim 2, in which said at least one plate (27;41;42;43;60) has a color contrasting with the color of said at least one of the interconnected elements (2,5,6) where said at least one recess (28;44;47;51) is formed.

4. A fixation device according to any of the preceding claims, in which at least one of the interconnected elements (2,5,6) is made of a material transparent to X-radiation.

5. A fixation device according to any of the preceding claims, in which said releasably securing means (30,31;46,46a;49,50;53,54) comprises at least one hole (30;46;49;53) formed in said at least one recess (28;44;47;51) and at least one pin (31;46a;50;54) formed integrally with said at least one plate (27;41;42;43;60) and fitted in said at least one hole (30;46;49;53).

6. A fixation device according to any of the preceding claims, in which said clamps (5,6) and said extendible central body (2) are made of a material transparent to X-radiation.

7. A fixation device according to Claim 6, whereby the at least one plate (27;42;60) is releasably secured in at least one recess (28;47) formed in the surface of said central body (2).

8. A fixation device according to Claim 7, in which said central body comprises two parts (8,9) telescoping slidable one on the other, each of said parts including a first portion (8a;9a) of elongate shape formed integrally with a second end portion (8b;9b) of substantially cylindrical shape.

9. A fixation device according to Claim 8, in which the first portions (8a,9a) of the parts (8,9) are slidably coupled to each other by means of a driving groove (7) formed longitudinally in one the first portion (8a) and a corresponding slide (7a) formed longitudinally in the other first portion (9a).

10. A fixation device according to Claim 9, whereby the plate (43) is releasably secured in a recess (51) of said slide (7a), said plate (43) consisting of an elongate bar bearing a calibrated scale (43a) impressed in a visible outward surface (43b) thereof to provide a measure of the extension of the central body (2) produced by a sliding movement of the slidably assembled parts (8,9).

11. A fixation device according to Claim 6, whereby the at least one plate (41) is releasably secured in at least one recess (44) formed in the surface of at least one of the clamps (5,6).

12. A fixation device according to any of the preceding claims, in which said at least one plate (27;41;42;43;60) has a multilineal contour shape.

13. A fixation device according to Claim 12, in which said multilineal contour is oval, circular, or comma-shaped.

14. A fixation device according to any of the preceding claims, wherein said at least one plate (27;41;42;43;60) is made of a plastic transparent to X-radiation.

15. A fixation device according to any of Claims 1 to 13, in which said at least one plate (27;41;42;43;60) is made of a material having a low interference with X-ray, preferably a plastic opaque to X-radiation or an aluminum alloy.

16. A fixation device according to any of the preceding claims, in which the thickness of said plate (27;41;42;43;60) is comprised in the range of 0.5 to 1.5 millimeters.

## Patentansprüche

1. Äußerliche Fixierungsvorrichtung zum Richten von Knochenbrüchen umfassend eine Stützstruktur (1; 40) von miteinander verbundenen Bauteilen (2, 5, 6), die einen ausziehbaren Mittelkörper (2) und Klemmen (5, 6) für Knochenschrauben umfassen, die an entsprechenden gegenüberliegenden Enden (3, 4) des Mittelkörpers (2) angeordnet sind, **dadurch gekennzeichnet, dass** die Vorrichtung mindestens eine Platte (27; 41; 42; 43; 60) mit vorherbestimmter Konturgestalt und Dicke, mindestens eine Ausnehmung (28; 44; 47; 51), die auf der Oberfläche mindestens eines der miteinander verbundenen Bauteile (2, 5, 6) ausgebildet ist und deren Konturgestalt der Konturgestalt der mindestens einen Platte (27; 41; 42; 43; 60) angepasst ist, und Mittel (30, 31; 46, 46a; 49, 50; 53, 54) zum lösbaren Sichern der mindestens einen Platte (27; 41; 42; 43; 60) in der mindestens einen Ausnehmung (28; 44; 47; 51) umfasst.

2. Fixierungsvorrichtung nach Anspruch 1, bei der die Tiefe der mindestens einen Ausnehmung (28; 44; 47; 51) im Wesentlichen gleich der Dicke der mindestens einen Platte (27; 41; 42; 43; 60) ist.

3. Fixierungsvorrichtung nach Anspruch 1 oder 2, bei der die mindestens eine Platte (27; 41; 42; 43; 60) eine Farbe aufweist, die sich von der Farbe des mindestens einen der miteinander verbundenen Bauteile (2, 5, 6) absetzt, dort wo die mindestens eine Ausnehmung (28; 44; 47; 51) ausgebildet ist.

4. Fixierungsvorrichtung nach einem der vorhergehenden Ans prüche, bei der mindestens eines der miteinander verbundenen Bauteile (2, 5, 6) aus einem für Röntgenstrahlung durchlässigem Material besteht.

5. Fixierungsvorrichtung nach einem der vorhergehenden Ans prüche, bei der das lösbare Sicherungsmittel (30, 31; 46, 46a; 49, 50; 53, 54) mindestens eine Bohrung (30; 46; 49; 53), die in der mindestens einen Aussparung (28; 44; 47; 51) ausgebildet ist, und mindestens einen Stift (31; 46a; 50; 54) umfasst, der einstückig mit der mindestens einen Platte (27; 41; 42; 43; 60) ausgebildet und in der mindestens einen Bohrung (30; 46; 49; 53) eingepasst ist.

6. Fixierungsvorrichtung nach einem der vorhergehenden Ans prüche, bei der die Klemmen (5, 6) und der ausziehbare Mittelkörper (2) aus einem für Röntgenstrahlung durchlässigen Material bestehen.

7. Fixierungsvorrichtung nach Anspruch 6, bei der die mindestens eine Platte (27; 42; 60) lösbar in der mindestens einen Ausnehmung (28; 47) gesichert ist, die in der Oberfläche des Mittelkörpers (2) ausgebildet ist.

8. Fixierungsvorrichtung nach Anspruch 7, bei der der zentrale Mittelkörper zwei teleskopisch aufeinander gleitbare Teile (8, 9) umfasst, die jeweils einen ersten Bereich (8a; 9a) mit lang gestreckter Gestalt beinhalten, der einstückig mit einem zweiten Endbereich (8b; 9b) mit im Wesentlichen zylindrischer Gestalt ausgebildet ist.

9. Fixierungsvorrichtung nach Anspruch 8, bei der die ersten Bereiche (8a, 9a) der Teile (8, 9) gleitbar miteinander verbunden sind mittels einer Verschiebungsnut (7), die in Längsrichtung in dem ersten Bereich (8a) ausgebildet ist, und eines entsprechenden Gleitstücks (7a), das in Längsrichtung in dem anderen ersten Bereich (9a) ausgebildet ist.

10. Fixierungsvorrichtung nach Anspruch 9, bei der die Platte (43) lösbar in einer Ausnehmung (51) des Gleitstücks (7a) festgelegt ist und aus einem lang gestreckten Stab besteht, der eine kalibrierte Skala (43a) trägt, die in einer sichtbaren nach außen gewandten Oberfläche (43b) dessen eingeprägt ist, um ein Messgerät für die Verlängerung des Mittelkörpers (2) bereit zu stellen, die durch eine gleitende Bewegung der gleitend angeordneten Teile (8, 9) hervorgerufen wird.

11. Fixierungsvorrichtung nach Anspruch 6, bei der die mindestens eine Platte (41) in der mindestens einen Ausnehmung (44) lösbar festgelegt ist, die in der Oberfläche mindestens einer der Klemmen (5, 6) ausgebildet ist.

12. Fixierungsvorrichtung nach einem der vorhergehenden Ans prüche, bei der die mindestens eine Platte (27; 41; 42; 43; 60) eine multilineare Konturgestalt aufweist.

13. Fixierungsvorrichtung nach Anspruch 12, bei der die multilineare Kontur oval, kreisförmig oder kommaförmig ist.

14. Fixierungsvorrichtung nach einem der vorhergehenden Ans prüche, bei der die mindestens eine Platte (27; 41; 42; 43; 60) aus einem für Röntgenstrahlung durchlässigen Kunststoff besteht.

15. Fixierungsvorrichtung nach einem der Ansprüche 1 bis 13, bei der die mindestens eine Platte (27; 41; 42; 43; 60) aus einem Material mit niedriger Interferenz für Röntgenstrahlen besteht, vorzugsweise einem für Röntgenstrahlung undurchlässigem Kunststoff oder einer Aluminiumlegierung besteht.

16. Fixierungsvorrichtung nach einem der vorhergehenden Ans prüche, bei der die Dicke der Platte (27; 41; 42; 43; 60) im Bereich zwischen 0,5 und 1,5 mm liegt.

## Revendications

1. Dispositif de fixation externe pour la réduction de fractures osseuses comportant une structure (1; 40) porteuse d'éléments connectés (2, 5, 6), lesdits éléments connectés (2, 5, 6) comportant un corps central extensible (2) et des clamps (5, 6) pour vis osseuses articulés respectivement sur les extrémités opposées (3,4) du corps central (2), **caractérisé en ce qu'**il comporte au moins une plaque (27; 41; 42; 43; 60) de forme périphérique et d'épaisseur prédéterminées, au moins un logement (28; 44; 47; 51) réalisé sur la surface d'au moins un des éléments connectés (2, 5, 6) et dont la forme périphérique est complémentaire avec celle d'au moins une plaque (27; 41; 42; 43; 60), et des moyens (30, 31; 46, 46a; 49, 50; 53, 54) de fixation réversible d'au moins une plaque (27; 41; 42; 43; 60) dans au moins un logement (28; 44; 47; 51).

2. Dispositif de fixation selon la revendication 1, **caractérisé en ce que** la profondeur d'au moins un logement (28; 44; 47; 51) est sensiblement égale à l'épaisseur d'au moins une plaque (27; 41; 42; 43; 60).

3. Dispositif de fixation selon la revendication 1 ou 2, **caractérisé en ce qu'**au moins une plaque (27; 41; 42; 43; 60) est de couleur contrastant avec la couleur de ledit au moins un des éléments connectés (2, 5, 6) où ledit au moins un logement (28; 44; 47; 51) est réalisé.

4. Dispositif de fixation selon l'une des revendications précédentes, **caractérisé en ce qu'**au moins un des éléments connectés (2, 5, 6) est réalisé au moyen d'un matériau transparent aux rayons X.

5. Dispositif de fixation selon l'une des revendications précédentes, **caractérisé en ce que** les moyens de fixation réversible (30, 31; 46, 46a; 49, 50; 53, 54) comportent au moins un perçage (30; 46; 49; 53) réalisé dans ledit au moins un logement (28; 44; 47; 51) et au moins un pion (31; 46a; 50; 54) solidaire de ladite au moins une plaque (27; 41; 42; 43; 60) et disposé dans ledit au moins un perçage (30; 46; 49; 53).

6. Dispositif de fixation selon l'une des revendications précédentes, **caractérisé en ce que** les clamps (5,6) et le corps central extensible (2) sont réalisés au moyen d'un matériau transparent aux rayons X.

7. Dispositif de fixation selon la revendication 6, **caractérisé en ce qu'**au moins une plaque (27; 42; 60) est fixée de manière dans au moins un logement (28, 47) réalisé sur la surface du corps central (2).

8. Dispositif de fixation selon la revendication 7, **caractérisé en ce que** le corps central comporte deux éléments (8, 9) coulissant téléscopiquement l'un sur l'autre, chacun de ces éléments comportant une première portion (8a;9a) de forme allongée solidaire d'une seconde portion (9a;9b) de forme sensiblement cylindrique.

9. Dispositif de fixation selon la revendication 8, **caractérisé en ce que** les premières portions (8a; 9a) des éléments (8, 9) sont reliées ensemble de sorte à réaliser une glissière, au moyen d'une rainure (7) de guidage réalisée longitudinalement dans une première portion (8a) et d'un coulisseau correspondant (7a) réalisé longitudinalement dans l'autre première portion (9a).

10. Dispositif de fixation selon la revendication 9, **caractérisé en ce que** la plaque (43) est fixée de manière réversible dans un logement (51) du coulisseau (7a), la plaque (43) étant une barrette allongée supportant une règle graduée (43a) imprimée sur une face extérieure visible de cette dernière afin de fournir une mesure de l'extension du corps central (2) réalisée par la translation des éléments coulissants (8, 9).

11. Dispositif de fixation selon la revendication 6, **caractérisé en ce qu'**au moins une plaque (41) est fixée de manière dans au moins un logement (44) réalisé sur la surface d'au moins un clamp (5, 6).

12. Dispositif de fixation selon l'une des revendications précédentes, **caractérisé en ce qu'**au moins une plaque (27; 41; 42; 43; 60) présente une forme périphérique multilinéaire.

13. Dispositif de fixation selon la revendication 12, **caractérisé en ce que** la forme périphérique multilinéaire est ovale, circulaire, ou en forme de virgule.

14. Dispositif de fixation selon l'une des revendications précédentes, **caractérisé en ce qu'**au moins une plaque (27; 41; 42; 43; 60) est réalisée en matière plastique transparent aux rayons X.

15. Dispositif de fixation selon l'une des revendications 1 à 13, **caractérisé en ce qu'**au moins une plaque (27; 41; 42; 43; 60) est réalisée en un matériau présentant de faibles interférences avec les rayons X, préférablement en matière plastique opaque aux rayons X ou en alliage d'aluminium.

16. Dispositif de fixation selon l'une des revendications précédentes, **caractérisé en ce que** l'épaisseur de la plaque (27; 41; 42; 43; 60) est comprise entre 0,5 mm et 1,5 mm.
